# EUROPEAN PATENT APPLICATION

(11) **EP 4 640 667 A1**
(43) Date of publication of application: **29.10.2025**
(21) Application number: 23906505.5
(22) Date of filing: 08.11.2023
(51) Int. Cl.: C07C 209/36, C07B 61/00, C07C 211/46

(54) **METHOD FOR PRODUCING HYDRIDE**

(30) Priority: 21.12.2022 JP 2022204813
(71) Applicant: Sumitomo Chemical Company, Limited, Tokyo 103-6020 (JP)
(72) Inventor: MURAKAMI, Masayoshi, Niihama-shi, Ehime 792-8521 (JP); YONEMOTO, Tetsuro, Ichihara-shi, Chiba 299-0195 (JP)
(74) Representative: Vossius & Partner Patentanwälte Rechtsanwälte mbB
(86) International application number: PCT/JP2023/040218
(87) International publication number: WO 2024/135134

(57) **Abstract**

A selectivity of a produced hydrogenated compound is further improved. Provided is a method for producing a hydrogenated compound, including a step of bringing a compound having a double bond and/or a triple bond into contact with a mixed gas containing an ammonia gas and a hydrogen gas to reduce the compound having a double bond and/or a triple bond into a hydrogenated compound.

## Description

### TECHNICAL FIELD

The present invention relates to a method for producing a hydrogenated compound, and more specifically to a method for producing a hydrogenated compound, in which a compound having a double bond and/or a triple bond is reduced with hydrogen to produce a hydrogenated compound.

### BACKGROUND ART

For example, a production method for producing aniline as a hydrogenated compound by reducing nitrobenzene as a raw material compound having a double bond with hydrogen to hydrogenate nitrobenzene is known (See Patent Document 1.). As a hydrogen source in such a method for producing aniline, a fossil fuel such as butane has been conventionally used.

In recent years, various attempts have been made to reduce the degree of dependence on a fossil fuel that can generate a greenhouse gas that promotes global warming. For example, a method for producing a hydrogenated compound using ammonia as a hydrogen source instead of a hydrogen source derived from a fossil fuel in performing hydrogenation as described in Patent Document 1 above is known (See Patent Document 2.).

### PRIOR ART DOCUMENTS

### PATENT DOCUMENTS

Patent Document 1: JP-A-49-231
Patent Document 2: JP-A-2014-181197

### SUMMARY OF THE INVENTION

### PROBLEMS TO BE SOLVED BY THE INVENTION

However, it may be difficult to say that a selectivity of an intended hydrogenated compound is sufficient in some methods for producing a hydrogenated compound according to prior art as described above.

### MEANS FOR SOLVING THE PROBLEMS

As a result of intensive studies to solve the above problem, the present inventors have found that the above problem can be solved by particularly using ammonia in hydrogenating a raw material compound, and have completed the present invention.

That is, the present invention provides the following [1] to [10].
[1] A method for producing a hydrogenated compound, including a step of bringing a compound having a double bond and/or a triple bond into contact with a mixed gas containing an ammonia gas and a hydrogen gas to reduce the compound having a double bond and/or a triple bond into a hydrogenated compound.
[2] The method for producing a hydrogenated compound according to [1], in which a concentration of the ammonia gas in the mixed gas is at least 0.01% by volume.
[3] The method for producing a hydrogenated compound according to [2], in which a concentration of the ammonia gas in the mixed gas is 0.05 to 2.5% by volume.
[4] The method for producing a hydrogenated compound according to any one of [1] to [3], in which the mixed gas further contains a nitrogen gas.
[5] The method for producing a hydrogenated compound according to any one of [1] to [4], in which the compound having a double bond and/or a triple bond is an aromatic compound.
[6] The method for producing a hydrogenated compound according to any one of [1] to [5], in which the compound having a double bond and/or a triple bond is at least one selected from the group consisting of phenol, benzene, and nitrobenzene.
[7] The method for producing a hydrogenated compound according to any one of [1] to [6], in which the hydrogenated compound is at least one selected from the group consisting of cyclohexanone, cyclohexanol, cyclohexane, cyclohexene, and aniline.
[8] The method for producing a hydrogenated compound according to any one of [1] to [7], in which the compound having a double bond and/or a triple bond is nitrobenzene, and the hydrogenated compound is aniline.
[9] The method for producing a hydrogenated compound according to any one of [1] to [8], in which the hydrogen gas is a hydrogen gas derived from ammonia.
[10] The method for producing a hydrogenated compound according to any one of [1] to [9], in which the ammonia gas is an ammonia gas derived from hydrogen produced using renewable energy and nitrogen.

### EFFECT OF THE INVENTION

According to the present invention, a selectivity of a produced hydrogenated compound can be further improved, and an intended hydrogenated compound can be more efficiently produced.

### MODE FOR CARRYING OUT THE INVENTION

### <Method for producing hydrogenated compound>

Hereinafter, a method for producing a hydrogenated compound according to the present embodiment will be specifically described.

The method for producing a hydrogenated compound according to the present embodiment includes a step of bringing a compound having a double bond and/or a triple bond into contact with a mixed gas containing an ammonia gas and a hydrogen gas to reduce the compound having a double bond and/or a triple bond into a hydrogenated compound (Hereinafter, also referred to as a contact and hydrogenation step.).

First, the "compound having a double bond and/or a triple bond" and the "mixed gas" applied to the method for producing a hydrogenated compound according to the present embodiment will be described.

### (1) Compound having a double bond and/or a triple bond

In the present embodiment, the "compound having a double bond and/or a triple bond" is not particularly limited as long as it is a compound having a structure including a double bond and/or a triple bond.

In the present embodiment, examples of the "compound having a double bond" as a raw material compound include a compound having one double bond, such as ethylene, propylene, butylene, isobutylene, pentene, cyclopentene, hexene, or cyclohexene, and a compound having two or more double bonds, such as pentadiene, hexadiene, heptadiene, benzene, nitrobenzene, or phenol.

In the present embodiment, examples of the "compound having a triple bond" as a raw material compound include acetylene and propyne.

In the present embodiment, examples of a suitable "compound having a double bond and/or a triple bond" include an aromatic compound. In the present embodiment, the "compound having a double bond and/or a triple bond" is preferably an aromatic compound.

In the present embodiment, examples of the "aromatic compound" include an aromatic carbocyclic compound optionally having a substituent and an aromatic heterocyclic compound optionally having a substituent, and these include a compound having a structure in which a plurality of ring structures are condensed. Here, the aromatic heterocyclic compound can include, in addition to a compound in which a heterocycle itself exhibits aromaticity, a compound in which an aromatic ring is condensed to a heterocycle that does not exhibit aromaticity.

Among the aromatic heterocyclic compounds, specific examples of a compound in which a heterocycle itself exhibits aromaticity include oxadiazole, thiadiazole, thiazole, oxazole, thiophene, pyrrole, phosphole, furan, pyridine, pyrazine, pyrimidine, triazine, pyridazine, quinoline, isoquinoline, carbazole, and dibenzophosphole.

Among the aromatic heterocyclic compounds, specific examples of a compound in which an aromatic ring is condensed to a heterocycle that does not exhibit aromaticity include phenoxazine, phenothiazine, dibenzoborole, dibenzosilole, and benzopyran.

Specific examples of the "aromatic compound" include phenol, benzene, and nitrobenzene. The "aromatic compound" is preferably at least one selected from the group consisting of benzene and nitrobenzene, and more preferably nitrobenzene.

Here, "optionally having a substituent" can include both a mode where each of hydrogen atoms constituting the compound or group has no substituent and a mode where some or all of one or more hydrogen atoms are replaced with substituents.

In the present embodiment, examples of the substituent include a halogen atom, an alkyl group, an aryl group, an alkoxy group, an aryloxy group, an alkylthio group, an arylthio group, a monovalent heterocyclic group, a substituted amino group, an acyl group, an imine residue, an amide group, an acidimide group, a substituted oxycarbonyl group, an alkenyl group, an alkynyl group, a cyano group, and a nitro group.

### (2) Hydrogenated compound

In the present embodiment, specific examples of the "hydrogenated compound" that can be produced include cyclohexanone, cyclohexanol, cyclohexane, cyclohexene, and aniline. The "hydrogenated compound" is preferably at least one selected from the group consisting of cyclohexane, cyclohexene, and aniline, and more preferably aniline.

In the method for producing a hydrogenated compound according to the present embodiment, as described above, the "compound having a double bond and/or a triple bond" is preferably nitrobenzene, and the "hydrogenated compound" is preferably aniline.

Here, for example, when the "compound having a double bond and/or a triple bond" is nitrobenzene and the "hydrogenated compound" that is an object of a "selectivity of hydrogenated compound" is aniline, a "by-product" is a product containing an intermediate product other than the hydrogenated compound, and an object of a "selectivity of by-product" is nitrosobenzene.

### (Contact and hydrogenation step)

In the method for producing a hydrogenated compound according to the present embodiment, as described above, the above-described compound having a double bond and/or a triple bond is brought into contact with a mixed gas containing an ammonia gas and a hydrogen gas to reduce the compound having a double bond and/or a triple bond into a hydrogenated compound.

When the compound having a double bond and/or a triple bond is brought into contact with the mixed gas, a property of the compound having a double bond and/or a triple bond is not particularly limited, and specifically, the compound may be in a gas state (gas phase) or a liquid state (liquid phase).

When the compound having a double bond and/or a triple bond is brought into contact with the mixed gas, implementation conditions such as a temperature condition and a pressure condition are not particularly limited, and can be any conventionally known suitable conditions corresponding to an intended hydrogenated compound.

As the implementation conditions in the method for producing a hydrogenated compound according to the present embodiment, for example, the temperature is preferably 50 to 400°C and the pressure is preferably 0 to 30 MPaG, and the temperature is more preferably 100 to 300°C and the pressure is more preferably 0 to 7 MPaG.

Specifically, for example, when the intended hydrogenated compound is aniline and the compound having a double bond and/or a triple bond is nitrobenzene, as the implementation conditions in the method for producing a hydrogenated compound according to the present embodiment, the temperature only needs to be preferably 175 to 370°C and the pressure only needs to be preferably 0 to 0.5 MPaG, and the temperature only needs to be more preferably 190 to 300°C and the pressure only needs to be more preferably 0 to 0.4 MPaG.

When the intended hydrogenated compound is cyclohexane and the compound having a double bond and/or a triple bond is benzene, specifically, the temperature only needs to be preferably 50 to 350°C and the pressure only needs to be preferably 1 to 20 MPaG, and the temperature only needs to be more preferably 100 to 250°C and the pressure only needs to be more preferably 2 to 7 MPaG.

When the intended hydrogenated compound is cyclohexene and the compound having a double bond and/or a triple bond is benzene, specifically, the temperature only needs to be preferably 50 to 250°C and the pressure only needs to be preferably 1 to 20 MPaG, and the temperature only needs to be more preferably 100 to 200°C and the pressure only needs to be more preferably 2 to 7 MPaG.

When the intended hydrogenated compound is cyclohexanone or cyclohexanol and the compound having a double bond and/or a triple bond is phenol, specifically, the temperature only needs to be preferably 100 to 220°C and the pressure only needs to be preferably 0 to 1 MPaG, and the temperature only needs to be more preferably 140 to 160°C and the pressure only needs to be more preferably 0 to 0.3 MPaG.

The contact and hydrogenation step according to the method for producing a hydrogenated compound according to the present embodiment can be performed using any conventionally known suitable reactor, specifically, for example, a heat exchange flow-type reactor or a batch-type reactor. In the contact and hydrogenation step, the reactor is preferably constituted by a vessel having pressure resistance, and as a material of the reactor, carbon steel or stainless steel is preferably used from a viewpoint of ensuring pressure resistance and corrosion resistance.

Hereinafter, the mixed gas that can be used in the method for producing a hydrogenated compound according to the present embodiment will be specifically described.

### (Mixed gas)

Preparation of the mixed gas in the method for producing a hydrogenated compound according to the present embodiment can be performed with any conventionally known suitable apparatus, for example, a cylinder or a pipe including a flow rate control valve.

In the method for producing a hydrogenated compound according to the present embodiment, the mixed gas containing an ammonia gas and a hydrogen gas to be brought into contact with the above-described compound having a double bond and/or a triple bond can further contain a gas in addition to the ammonia gas and the hydrogen gas.

Hereinafter, the gas that can be contained in the mixed gas in the method for producing a hydrogenated compound according to the present embodiment will be specifically described.

### (1) Ammonia gas

In the method for producing a hydrogenated compound according to the present embodiment, a concentration of the ammonia gas in the mixed gas is preferably at least 0.01% by volume, more preferably 0.01 to 10% by volume, still more preferably 0.02 to 7.5% by volume, particularly preferably 0.03 to 5.0% by volume, and further particularly preferably 0.05 to 2.5% by volume from a viewpoint of further improving a selectivity of an intended hydrogenated compound.

In the method for producing a hydrogenated compound according to the present embodiment, an origin of the ammonia gas contained in the mixed gas is not particularly limited. The ammonia gas contained in the mixed gas may be, for example, a biomass-derived ammonia gas (ammonia). Furthermore, the ammonia gas is preferably an ammonia gas derived from nitrogen and hydrogen produced using renewable energy, or derived from nitrogen and hydrogen produced by a decomposition step of decomposing a fossil fuel at high temperature, the hydrogen being produced while recovering and storing carbon dioxide or carbon generated in the decomposition step, from a viewpoint of further reducing an environmental load.

Here, the "hydrogen produced using renewable energy" refers to hydrogen (gas) produced by any conventionally known suitable method, specifically, for example, by electrolyzing water (H₂O) using electric power obtained by, for example, solar power generation, wind power generation, geothermal power generation, or thermal power generation using a biomass fuel.

In addition, the "hydrogen produced by a decomposition step of decomposing a fossil fuel at high temperature, the hydrogen being produced while recovering and storing carbon dioxide or carbon generated in the decomposition step" refers to, for example, hydrogen (gas) produced by using any conventionally known suitable method such as steam reforming or thermal decomposition of liquefied natural gas (LNG) or methane, the hydrogen (gas) being produced while recovering generated carbon dioxide or carbon by any conventionally known suitable method, specifically, for example, a chemical absorption method using an amine or the like, and burying the carbon dioxide or carbon in the ground, or hydrogen (gas) produced by directly thermally decomposing methane while separating, recovering, and storing (solid) carbon.

The ammonia gas (ammonia) used in the method for producing a hydrogenated compound according to the present embodiment can be produced by any conventionally known suitable production method, for example, by bringing "hydrogen produced using renewable energy or produced by a decomposition step of decomposing a fossil fuel at high temperature, the hydrogen being produced while recovering and storing carbon dioxide or carbon generated in the decomposition step" into direct contact with nitrogen as described above while a predetermined catalyst coexists.

### (2) Hydrogen gas

In the method for producing a hydrogenated compound according to the present embodiment, a concentration of the hydrogen gas in the mixed gas is preferably 50 to 99.999% by volume, more preferably 75 to 99.99% by volume, and still more preferably 75 to 99.95% by volume from a viewpoint of further improving a reaction rate of an intended hydrogenated compound.

In the method for producing a hydrogenated compound according to the present embodiment, the hydrogen gas contained in the mixed gas is preferably a hydrogen gas derived from ammonia from a viewpoint of further reducing an environmental load.

Here, examples of the "hydrogen gas derived from ammonia" include a hydrogen gas produced by any conventionally known suitable ammonia reforming.

Specifically, the ammonia reforming can be performed, for example, using any conventionally known suitable ammonia reforming catalyst containing at least one metal selected from the group consisting of Fe, Co, Ni, Mo, and Mn or at least one noble metal selected from the group consisting of Pt, Pd, Ir, Rh, and Ru, and preferably using a reactor having any conventionally known suitable configuration including a heating apparatus because the decomposition reaction of ammonia is an endothermic reaction.

Note that hydrogen (gas) produced by ammonia reforming inevitably contains unreacted ammonia. When hydrogen produced by ammonia reforming is applied to, for example, a method for producing a hydrogenated compound, ammonia inevitably contained is removed by any conventionally known suitable purification method.

However, according to the method for producing a hydrogenated compound according to the present embodiment, since an ammonia gas is contained in the mixed gas, even when a "hydrogen gas derived from ammonia" produced by, for example, ammonia reforming as described above is used, ammonia (gas) that can be inevitably contained can be effectively used as a component of the mixed gas for producing a hydrogenated compound without intentionally performing a step of removing ammonia from hydrogen produced by ammonia reforming.

Therefore, according to the method for producing a hydrogenated compound according to the present embodiment, even when a "hydrogen gas derived from ammonia" produced by ammonia reforming is used, it is not necessary to perform a step of removing ammonia that is inevitably contained. Therefore, a hydrogenated compound can be more easily produced, and furthermore, capital investment for such a step is not necessary, and therefore production cost of a hydrogenated compound can be further reduced.

### (3) Nitrogen gas

In the present embodiment, the mixed gas may further contain a nitrogen gas in addition to the ammonia gas and the hydrogen gas from a viewpoint of further reducing production cost of a hydrogenated compound.

In the present embodiment, a concentration of the nitrogen gas in the mixed gas is preferably 0.001 to 50% by volume, more preferably 0.01 to 25% by volume, and still more preferably 0.1 to 1% by volume from a viewpoint of further reducing production cost and further improving a reaction rate of a hydrogenated compound.

In the method for producing a hydrogenated compound according to the present embodiment, when the above-described compound having a double bond and/or a triple bond is brought into contact with the above-described mixed gas, it is preferable to bring the compound having a double bond and/or a triple bond into contact with the mixed gas in a state where a catalyst coexists (in the presence of a catalyst) from a viewpoint of more efficiently producing an intended hydrogenated compound.

Here, the catalyst that can be used in the method for producing a hydrogenated compound according to the present embodiment will be specifically described.

As the catalyst that can be suitably applied to the method for producing a hydrogenated compound according to the present embodiment, any conventionally known suitable catalyst corresponding to an intended hydrogenated compound and a selected "compound having a double bond and/or a triple bond" can be used in a suitable mode.

Examples of the catalyst that can be suitably applied to the method for producing a hydrogenated compound according to the present embodiment include a cobalt-based catalyst, a copper-based catalyst, a nickel-based catalyst, a sponge nickel-based catalyst, a copper chromium-based catalyst, a copper iron alumina-based catalyst, a copper silicon-based catalyst (Cu-Si catalyst), a platinum-based catalyst, a palladium catalyst, and a ruthenium-based catalyst. As such a catalyst, any suitable catalyst available on the market can be selected and used.

In the method for producing a hydrogenated compound according to the present embodiment, when the catalyst is applied, any conventionally known suitable carrier such as silica gel or alumina and a diluent such as silicon carbide corresponding to a selected catalyst and a mode of a reactor can be further used as a preferable mode.

In the method for producing a hydrogenated compound according to the present embodiment, in addition to the above-described "contact and hydrogenation step", for example, any conventionally known suitable purification step for increasing a purity of an intended hydrogenated compound may be performed.

### EXAMPLES

Hereinafter, Examples according to the present invention will be described. The present invention is not limited to the following Examples.

### (Example 1)

### <Production of aniline>

0.5 g of a Cu-Si catalyst (E55W manufactured by JGC Catalysts and Chemicals Ltd.) and 1.5 g of silicon carbide (SHINANO-RUNDUM manufactured by Shinano Electric Refining Co., Ltd.) as a diluent were mixed and filled into a quartz reaction tube having an inner diameter of 10 mm to form a reaction bed.

The temperature of the reaction bed was set to 175°C, and a reduction treatment was performed for 30 minutes at a hydrogen gas flow amount (flow rate) of 90 mL/min and a nitrogen gas flow amount (flow rate) of 86 mL/min.

Next, a raw material gas generated by supplying nitrobenzene (liquid) as a raw material at 0.06 mL/min to a mixed gas obtained by joining a hydrogen gas at 60 mL/min, a nitrogen gas containing 0.2% by volume of ammonia gas at 100 mL/min, and a nitrogen gas at 220 mL/min was caused to flow through the reaction tube at normal pressure, and brought into contact with the reaction bed at a reaction temperature of 210°C in the reaction bed, thereby producing aniline. A composition of the raw material gas is also presented in Table 1 below.

Subsequently, the reaction gas derived from the reaction tube after being brought into contact with the reaction bed was cooled and liquefied, and thereby recovered as a liquid. The recovered liquid was analyzed by gas chromatography, and a conversion ratio (mol%) of nitrobenzene and a selectivity (mol%, carbon base) of a product (aniline and nitrosobenzene as a by-product) were calculated. The results (conversion ratio, selectivity of aniline (aniline selectivity), and selectivity of nitrosobenzene (nitrosobenzene selectivity)) are presented in Table 1 below.

### (Examples 2 and 3)

### <Production of aniline>

Aniline was produced in a similar manner to Example 1 except that the flow amounts (flow rates) of the ammonia gas and the nitrogen gas were as presented in Table 1 below. A composition of a raw material gas and results are presented in Table 1 below.

### (Comparative Example 1)

### <Production of aniline>

Aniline was produced in a similar manner to Example 1 except that an ammonia gas was not used and the other conditions were as presented in Table 1 below. A composition of a raw material gas and results are presented in Table 1 below.

### (Comparative Example 2)

### <Production of aniline>

Aniline was produced in a similar manner to Example 1 except that the flow amounts (flow rates) of the ammonia gas and the nitrogen gas were as presented in Table 1 below.

### [Table 1]

**(Table 1)**

| | Hydro gen | Nitro gen | Ammo nia | Ammonia concentr ation in mixed gas | Conver sion ratio | Aniline selecti vity | Nitrosobe nzene selectivi ty | Hydrogen concentr ation |
|---|---|---|---|---|---|---|---|---|
| | ml/mi n | ml/mi n | ml/m in | % by volume | % | % | % | % by volume |
| Example 1 | 60 | 308.3 | 0.18 | 0.05 | 49 | 99.86 | 0.03 | 16.28 |
| Example 2 | 60 | 307.9 | 0.6 | 0.17 | 56 | 99.90 | 0.02 | 16.28 |
| Example 3 | 60 | 299.2 | 9.3 | 2.5 | 49 | 99.86 | 0.03 | 16.28 |
| Compara tive Example 1 | 60 | 308.5 | 0 | 0 | 50 | 99.83 | 0.10 | 16.28 |
| Compara tive Example 2 | 60 | 215.5 | 93 | 25 | - | - | - | 16.28 |

As is apparent from Table 1 above, it was found that according to Examples 1 to 3 in which an ammonia-containing raw material gas was used, the selectivity of aniline which is an intended hydrogenated compound (aniline selectivity) could be effectively improved, and the selectivity of nitrosobenzene which is a by-product (nitrosobenzene selectivity) could be remarkably reduced as compared with Comparative Example 1 in which ammonia was not used. In addition, as presented in Table 1 above, in Comparative Example 2 in which the concentration of an ammonia gas in the mixed gas exceeded a predetermined range, as a result of falling of the reaction temperature in the reaction bed to the reactor temperature, production of aniline by allowing the reaction to proceed was not possible.

## Claims

1. A method for producing a hydrogenated compound, comprising a step of bringing a compound having a double bond and/or a triple bond into contact with a mixed gas containing an ammonia gas and a hydrogen gas to reduce the compound having a double bond and/or a triple bond into a hydrogenated compound.

2. The method for producing a hydrogenated compound according to claim 1, wherein a concentration of the ammonia gas in the mixed gas is at least 0.01% by volume.

3. The method for producing a hydrogenated compound according to claim 2, wherein a concentration of the ammonia gas in the mixed gas is 0.05 to 2.5% by volume.

4. The method for producing a hydrogenated compound according to claim 1 or 2, wherein the mixed gas further contains a nitrogen gas.

5. The method for producing a hydrogenated compound according to claim 1 or 2, wherein the compound having a double bond and/or a triple bond is an aromatic compound.

6. The method for producing a hydrogenated compound according to claim 1 or 2, wherein the compound having a double bond and/or a triple bond is at least one selected from the group consisting of phenol, benzene, and nitrobenzene.

7. The method for producing a hydrogenated compound according to claim 1 or 2, wherein the hydrogenated compound is at least one selected from the group consisting of cyclohexanone, cyclohexanol, cyclohexane, cyclohexene, and aniline.

8. The method for producing a hydrogenated compound according to claim 1 or 2, wherein the compound having a double bond and/or a triple bond is nitrobenzene, and the hydrogenated compound is aniline.

9. The method for producing a hydrogenated compound according to claim 1 or 2, wherein the hydrogen gas is a hydrogen gas derived from ammonia.

10. The method for producing a hydrogenated compound according to claim 1 or 2, wherein the ammonia gas is an ammonia gas derived from hydrogen produced using renewable energy and nitrogen.
